# EUROPEAN PATENT SPECIFICATION

(11) **EP 0 730 478 B1**
(45) Date of publication and mention of the grant of the patent: **16.04.2003**
(21) Application number: 94902389.9
(22) Date of filing: 24.11.1993
(51) Int. Cl.: A61M 29/00, A61F 2/06

(54) **A STENT**
STENT
EXTENSEUR

(43) Date of publication of application: 11.09.1996
(73) Proprietor: NITINOL MEDICAL TECHNOLOGIES, INC., Boston, MA 02210 (US)
(72) Inventor: SIMON, Morris, A., Boston, MA 02215 (US); KLESHINSKI, Stephen, J., Scituate, MA 02066 (US); RABKIN, Dmitry, Bookline, MA 02146 (US)
(74) Representative: Wilhelms, Rolf E., Dr.
(86) International application number: US9311441
(87) International publication number: WO95014500

(56) References cited:
- EP-A- 0 045 627
- WO-A-93/17636
- US-A- 4 512 338
- US-A- 4 739 762
- US-A- 4 830 003
- US-A- 5 123 917
- US-A- 5 224 953

## Description

### BACKGROUND OF THE INVENTION

### FIELD OF THE INVENTION

This invention relates to stents and is directed more particularly to a self-expanding, generally cylindrical stent which is repositionable after being set in place.

### BRIEF DESCRIPTION OF THE PRIOR ART

Self-expanding stents are generally known in the art. U.S. Patent No. 4,580,568, issued April 8, 1986, to Cesare Gianturco, discloses an endovascular stent formed of stainless steel wire. The stent is compressed into a reduced size having an outer diameter substantially smaller than the stent in its expanded shape. The stent is held in its compressed state during its passage through a small bore catheter until delivered into a vascular system passageway, whereupon the stress in the stent causes the stent to expand in the larger bore vascular passageway to hold open the passageway. When the stent is compressed, the bends in the wire, which is of a zig-zag configuration, store stress, and the stent is expandable by the release of the stress stored in the bends. Once set in place, the radial extremities of the stent bear against the inside walls of the passageway. There is no ready means by which the stent may be again compressed, or softened, so that the stent may be repositioned.

From US-A-5 224 953 a stent according to the general part of claim 1 is known, whereas WO-A-93/17636 discloses in elastomeric film connected to a stent, the stent beeing made of stainless steel, whereby the film does not serve the purpose to urge a stent made of thermal shape memory material upon cooling to a smaller configuration than the stent in its expanded condition.

It would be beneficial to the medical arts to have available a stent adapted for compression into a small size to facilitate introduction into a vascular passageway, and adapted for self-expansion in the vascular passageway to hold open the passageway, and also adapted to be softened and/or contracted to permit repositioning of the stent.

### SUMMARY OF THE INVENTION

It is, therefore, an object of the invention to provide a stent adapted to assume a first configuration in which the stent is expanded, capable of exercising considerable stress if confined, as by a vessel wall, and substantially tubular in configuration for holding open a vascular passageway, and a second configuration in which the stent is flexible, in a reduced stress state, and adapted to be compressed into a small enough size to fit within the small bore of a delivery catheter.

A further object of the invention is to provide such a stent which is adapted to change from the first condition of relative rigidity to the second condition of flexibility and reduced stress, by exposure to a preselected transition temperature, such that the stent may be relaxed in place in a vascular passageway by cooling to facilitate repositioning thereof without damage to walls of the passageway.

A still further object of the invention is to provide such a stent laminated within an elastomeric sleeve, the sleeve being expandable to conform to the stent's first, i.e. rigid, condition and having therein a bias towards assuming a smaller size, such that upon the stent's assuming the second, i.e. flexible, condition, the sleeve operates to compress the stent to a size less than its expanded size.

The object of the invention is solved by the stent according to claim 1; further preferred embodiments are covered by the claims 2 to 6.

The features of the invention, including various novel details of construction and combinations of parts, will now be more particularly described with reference to the accompanying drawings and pointed out in the claims. It will be understood that the particular devices embodying the invention are shown by way of illustration only and not as limitations of the invention. The principles and features of this invention may be employed in various and numerous embodiments without departing from the scope of the invention.

### BRIEF DESCRIPTION OF THE DRAWINGS

Reference is made to the accompanying drawings in which are shown illustrative embodiments of the invention, from which its novel features and advantages will be apparent.

In the drawings:
Fig. 1 is a perspective view of one form of stent illustrative of an embodiment of the invention;
Fig. 2 is a side elevational view thereof;
Fig. 3 is a side elevational view of an alternative embodiment thereof;
Fig. 4 is a side elevational view of a second alternative embodiment thereof;
Fig. 5 is a side elevational view of the stent shown in Fig. 1, but shown in a compressed condition;
Fig. 6 is a side elevational view of the stent shown in Figs. 1 and 2 with an elastomeric sleeve thereon;
Figs. 7A-7C are illustrative stylized diagrammatic views of one manner of use of the inventive devices of Figs. 1-6, as in the treatment of an aneurysm of a large artery;
Figs. 8A-8C are stylized diagrammatic views illustrative of another manner of use of the inventive device of Figs. 1-6, as in the treatment of compression or narrowing of a vessel; and
Figs. 9A-9E are stylized diagrammatic views illustrative of a manner of repositioning the inventive device of Figs. 1-6.

### DESCRIPTION OF THE PREFERRED EMBODIMENT

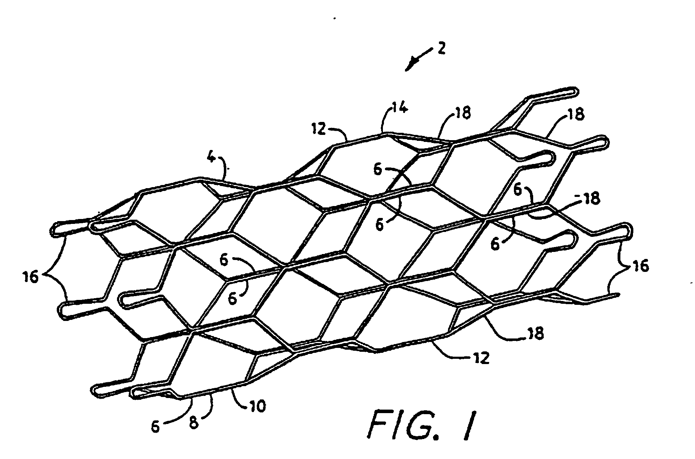

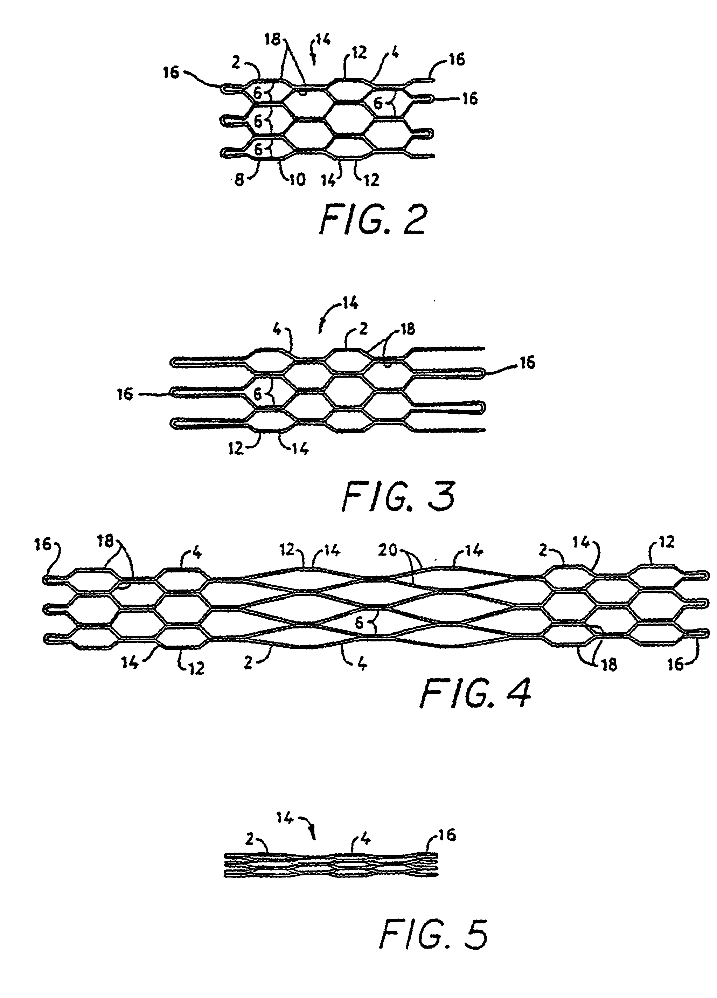

Referring to Figs. 1 and 2, it will be seen that an illustrative stent includes a skeletal frame 2, preferably formed from a single wire 4. The wire 4 includes a plurality of abutting straight portions 6 which are joined to each other, as by welding.

In Figs. 1 and 2, the illustrative stent is shown in a first condition in which the frame 2 is expanded, relatively rigid, and substantially tubular in configuration. Ends 8, 10 of the single wire 4 are disposed in one of the welded straight portions 6, such that there are no exposed wire free ends. disposed within or extending from the frame 2. The abutting and elongated straight portions 6 of the wire 4 facilitate the use of strong elongated welds to securely join the wire portions 6 together. The wire 4 preferably is round in cross-section. In the frame straight portions 6 the joined wire segments are disposed, relative to the tubular configuration of the frame, circumferentially thereof. The wire 4 abuts itself only at the straight portions 6 and does not cross itself at any point. Accordingly, the frame walls, that is, walls 12 of a tubular body portion 14 of the frame 2 have a thickness equal to the diameter of the wire 4.

The stent includes the body portion 14 and finger portions 16 extending generally axially from one, or both, ends of the body portion. The fingers facilitate a gradual reduction in radially outwardly extending pressure exerted by the stent on the wall of a vascular passageway in which the stent is located. Such gradual reduction of pressure facilities acceptance of the stent by the passageway and reduces deleterious reactions by the passageway wall to the presence of the stent. Referring to Fig. 3, it will be seen that the finger portion 16 may be extended further axially to lessen the probability of adverse reaction by the passageway wall to the pressure exerted against the wall by the stent frame 2.

The tubular body portion 14 comprises a mesh formed by the wire 4, the mesh comprising a plurality of interconnected cells 18 of a polygonal configuration when viewed in plan, providing straight sides to form the aforementioned straight portions 6. The polygonal cells 18 preferably are of a hexagonal configuration, which readily provides expansion and rigidity characteristics desirable in the structure and operation of the device. Preferably, the stent comprises six of the polygonal cells 18 circumferentially and an even number of the polygonal cells along its length, thereby facilitating formation of the stent by the single wire 4. The portion of the stent having the mesh construction exercises a substantially greater radial bias than do the finger portions 16. Thus, when it is desired to have more force near the ends of the stent than at its center, the embodiment shown in Fig. 4 may be used. Referring to Fig. 4, it will be seen that in this embodiment, the central portion of the tubular body portion 14 includes elongated cells 20 exercising less radial force than the cells 18.

The stent preferably is made of an alloy of nickel and titanium which provides the stent with a thermal memory. The unique characteristic of this alloy, known generally as "Nitinol", is its thermally triggered shape memory, which allows the stent constructed of the alloy to be cooled and thereby softened for loading into a catheter in a relatively compressed and elongated state, and regain the memoried shape when warmed to a selected temperature, such as human body temperature. The two interchangeable shapes are possible because of the two distinct micro-crystalline structures that are interchangeable with a small variation in temperature. The temperature at which the stent assumes its first configuration may be varied within wide limits by changing the composition of the alloy. Thus, while for human use the alloy may be focused on a temperature of 98.6°F for assumption of the first condition, the alloy readily may be modified for use in animals with different body temperatures.

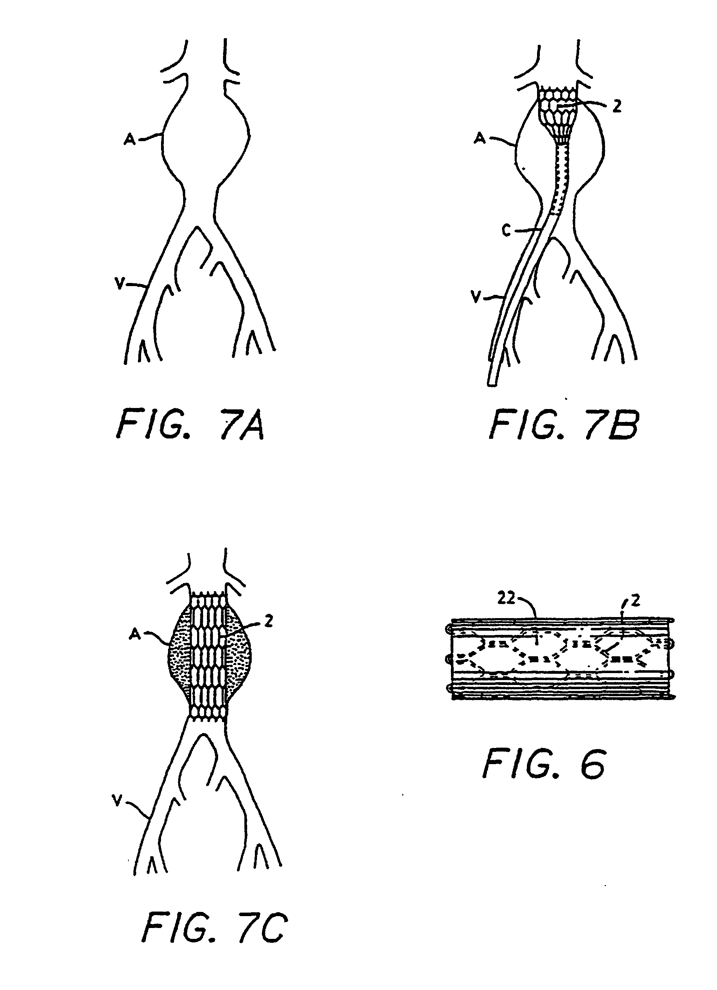

Accordingly, when the stents shown in Figs. 1-4 are subjected to a temperature at or less than the transition temperature, the relativity rigid stent changes to a second condition in which it is flexible, of reduced stress and collapsible. The stent does not, of its own accord, collapse, or compress, but the stent does become quite pliable, collapsible and compressible. By mechanical means, the stent may be compressed to a point at which the walls 12 of the body portion 14 of the stent frame 2 are positioned against each other, to form a stent diameter substantially equal to the combined thickness of the frame walls in abutting engagement with each other. In Fig. 5, the stent is shown approaching, but not yet having reached such minimal stent diameter. In the compressed condition. the stent is readily contained by a catheter C (Fig. 7B).

In Fig. 6, there is shown the stent according to the invention. As noted above, in the second condition of the stent, the stent becomes flexible and compressible, but does not of its own accord compress. According to Fig. 6, the stent body portion has disposed thereon an elastomeric sleeve 22. The sleeve 22 is expandable on the frame 2 as the frame expands to its enlarged configuration. However, as the sleeve expands, the sleeve exerts a compressive force on the frame. Upon cooling of the stent to or below the transition temperature, the stent becomes flexible and the compressive sleeve 22 urges the frame 2 to a third configuration of smaller diameter than the first configuration. Accordingly, upon cooling of the sleeved embodiment, the flexible frame automatically reduces in size, thereby rendering any repositioning of the stent, as by a grasping tool or other instrument, known in the art (not shown), a relatively simple matter. Again, upon removal of the cooling medium, the sleeved stent returns to its expanded condition.

The sleeved stent has an added benefit in that while an unsleeved stent will suffice in many instances, there are occasions when the affected passageway wall is in such a weakened condition that the provision of a new wall, or a graft, is required. The sleeved stent is essentially a graft and operates to provide a new passageway wall when required.

In operation, the sleeved stent is carried through an affected vascular passageway V (Fig. 7A) by the catheter C (Fig. 7B), which is of a thermally insulative material. At room temperature, and while cooled by infusion of a cool solution within the catheter, the stent remains in the second condition, flexible and of low stress. Being of low stress, the stent exercises negligible radial force against the inside wall of the catheter and is easily moved through the catheter at the appropriate time.

As the catheter enters the passageway V, the thermal insulative properties of the catheter and the flow of cool solution maintain the stent at less than body temperature. When the distal end of the catheter is properly disposed, as for example, in the vicinity of an aneurysm A (Fig. 7B), the stent is moved out of the end of the catheter C. As the stent contacts blood flow, and is subjected to body temperature, the exposed stent immediately and rapidly assumes its first condition, expanding against the walls of the passageway. Upon total ejection of the stent, the catheter is removed, leaving the stent in place to act as an internal wall graft (Fig. 7C).

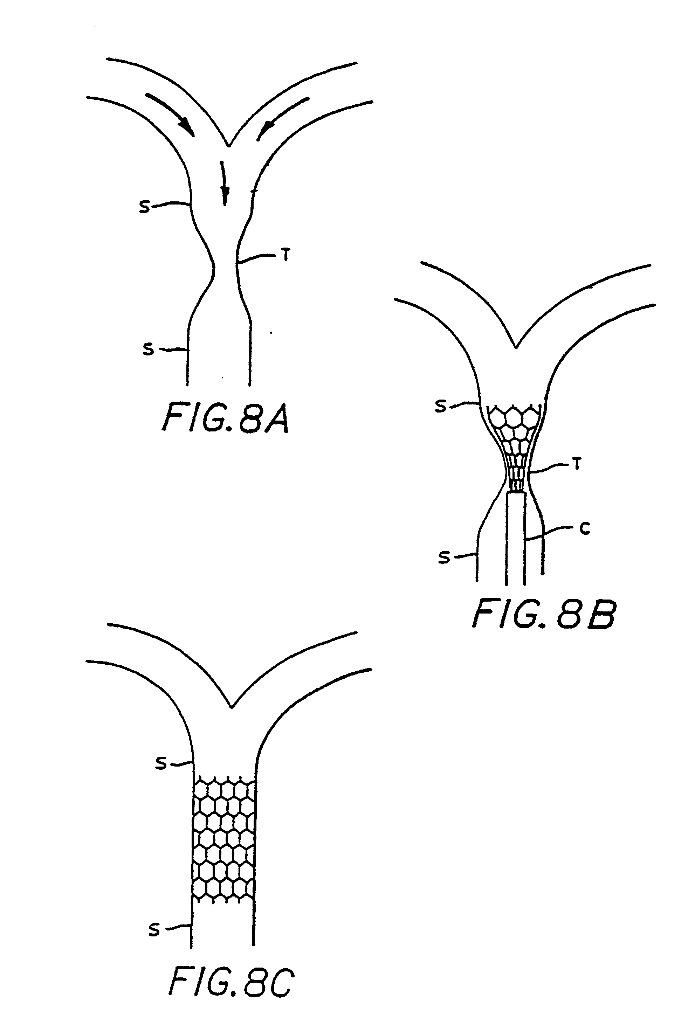

Referring to Figs. 8A-8C, it will be seen that in treatment of compression of a large vessel, such as a superior vene cava S, the catheter C (Fig. 8B) is moved through the vessel S to a point adjacent a stricture T. The stent 2 is moved from the catheter C, while the catheter is withdrawn, to place the emerging stent within the vessel and in the area of the stricture (Fig. 8B). As the stent emerges from the catheter, the stent, as it is exposed to the blood stream, assumes its first condition. Upon total removal of the stent from the catheter, the stent in its entirety is expanded against the wall of the vessel (Fig. 8C) to maintain the vessel in a free-flowing configuration.

The ratio of expanded stent diameter to compressed stent diameter can be controlled within limits by selection of wire diameter. The diameter of the expanded stent generally is on the order of 6 to 10 times the diameter of the compressed stent. In general, the greater the diameter of the wire 4, the less the ratio of the stent collapsed/expanded diameter. By selection of wire diameter, it is possible to vary the radial force which the expanded stent will exert on the interior walls of the passageway in which the stent is set.

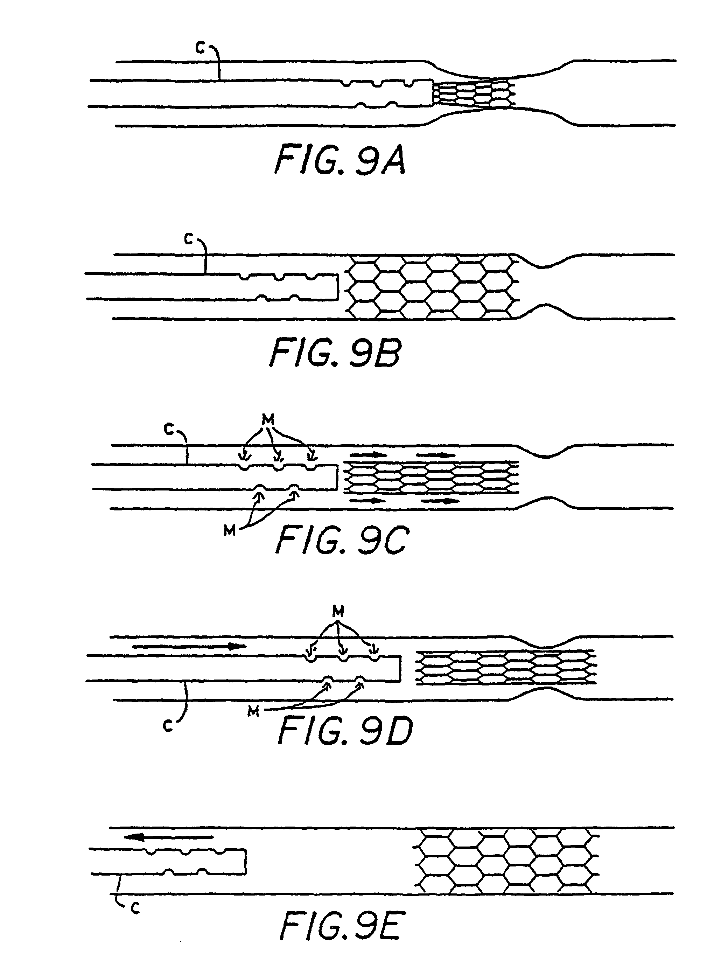

It is sometimes the case that once the stent is in place and in part expanded, it is recognized that the stent is somewhat off target (Figs. 9A and 9B) and requires repositioning. To reposition the stent of the present invention, the operator introduces into the passageway a cool medium M (Figs. 9C and 9D), such as a saline solution, having a temperature at or less than the transition temperature. When the cool solution encounters the stent, the stent immediately turns flexible and surrenders radial force against the passageway walls. In such relaxed state, the stent, which has no free wire ends, is easily slid into the proper position by manipulation of the catheter C (Fig. 9D), whereupon the flow of cool solution is stopped and the stent, upon returning to a body temperature, reassumes its expanded condition in the passageway (Fig. 9E). The catheter C is then withdrawn from the stent and from the passageway.

Thus, there is provided a stent which may be alloyed to have a selected temperature at which the stent assumes its first condition and a selected transition temperature, at which the stent assumes its second condition, and which includes a wire frame, wherein the diameter of the wire is selectable to provide a selected degree of expansion force. The stent is compressible to less than a catheter-size diameter to facilitate delivery of the stent to a location within a body passageway by a catheter. The stent may be sleeved or unsleeved. The stent is self-expanding upon delivery from the catheter and introduction to a body temperature, to provide an internal graft or hold open a passageway. Even after such positioning and expansion, the stent is rendered flexible and readily repositionable merely by the flow of a cool medium through the stent. And, finally, by termination of the flow of cool fluid, the stent automatically reassumes its passageway supporting rigid condition. Any required subsequent repositioning can be accomplished in the same manner.

It is to be understood that the present invention is by no means limited to the particular constructions herein disclosed and/or shown in the drawings, but also comprises any modifications or equivalents within the scope of the claims. For example, while the use of the stent has been illustrated in connection with the vascular system, it will be apparent to those skilled in the art that the stent herein shown and described finds equal utility in other bodily passageways.

## Claims

1. A stent for insertion in a body vessel including an elongate tubular body member (14) having a longitudinal axis, a first end, a second end, and an elongate chamber extending through said body member between said first and second ends (16), the body member (14) being formed of thermal shape memory material which is relatively pliable at temperatures below a transition temperature to permit said body member (14) to be collapsed toward said longitudinal axis to a collapsed configuration for insertion in said body vessel, said thermal shape memory material operating to expand said body member (14) radially outward from said collapsed configuration toward an expanded memory configuration in response to temperatures at or above said transition temperature to contact and apply radial force to said body vessel, said body member (14) including a skeletal frame (2) formed of said thermal shape memory material to define said elongate chamber, the skeletal frame including a plurality of interconnected open cells (18, 20) each of which includes two substantially parallel, spaced, straight side portions (6) which are substantially parallel to said longitudinal axis during said collapsed configuration, said expanded memory configuration and movement therebetween and endwall means extending between said side portions (6) at an angle to said longitudinal axis during said expanded memory configuration, said cells (18,20) being joined together only along the lengths of said straight side portions, **characterised in that** the stent comprises an elastomeric sleeve disposed on the stent and expandable therewith to conform to the stent's expanded condition, the sleeve having a bias exerting a compressive force on the stent, such that upon cooling of the sent below a selected transition temperature, the sleeve urges the flexible stent to a configuration smaller than the stent in its expanded condition.

2. The stent according to claim 1 wherein each cell (20) in said first area of said skeletal frame includes two substantially parallel spaced straight side portions (6) which are spaced apart for a distance substantially equal to the distance between the substantially parallel spaced side portions of the cells (18) in said second area of said skeletal frame, the endwall means of the cells (20) in said first area being greater in length than the endwall means of the cells (18) in said second area.

3. The stent according to claim 1 wherein an elastomeric sleeve is (22) disposed around said elongate body member (14) in contact with the straight side portions (6) of said cells (18, 20) and operates to expand and collapse with the body member (14), said elastomeric sleeve (22) operating when said skeletal frame (2) assumes said first expanded configuration to exert a compressive force on said skeletal frame (2) in said first expanded configuration and operating when said skeletal frame (2) is at or below said transition temperature to collapse said skeletal frame (2) to the second collapsed configuration, said straight side portions (6) and endwall means being configured to maintain said side portions (6) parallel to said longitudinal axis in said first expanded configuration, in said second collapsed configuration and during movement therebetween, said endwall means extending between said side portions (6) at an angle to said longitudinal axis in said first expanded condition and being forced to ward said longitudinal axis by said elastomeric sleeve (22) when said skeletal frame is at or below said transition temperature.

4. The stent according to claim 3 wherein said first area of said skeletal frame (2) includes cells (20) which are larger than the cells (18) in said second area of said skeletal frame (2) said cells (18, 20) in said first and second areas expanding at temperatures above said transition temperature to apply a radial force against said elastomeric sleeve (22) with the radial force applied by the cells (20) in said first area being less than the radial force applied by the cells (18) in said second area.

5. The stent according to claim 1 wherein the cells (18, 20) in said first and second areas of said skeletal frame (2) are polygonal in configuration, **characterized by** the endwalls of the cells (20) in said first area of the skeletal frame (2) being at a greater angle to the longitudinal axis of said body member (14) than the endwalls of the cells (18) in the second area of said skeletal frame (2) when said body member (14) is in the expanded memory configuration so that the cells (20) in the first area apply less radial force to the vessel than do the cells (18) in the second area.

6. The stent according to claim 5 wherein an elastomeric sleeve (22) is disposed around said elongate body member (14) in contact with the straight side portions (6) of said cells (18, 20) and operates to expand and collapse with the body member (14), said elastomeric sleeve (22) operating when said skeletal frame (2) assumes said first expanded configuration to exert a compressive force on said skeletal frame (2) in said first expanded configuration and operating when said skeletal frame (2) is at or below said transition temperature to collapse said skeletal frame (2) to the second collapsed configuration, said straight side portions (6) and endwall means being configured to maintain said side portions (6) parallel to said longitudinal axis in said first expanded configuration, in said second collapsed configuration and during movement therebetween, said endwall means extending between said side portions (6) at an angle to said longitudinal axis in said first expanded condition and being forced toward said longitudinal axis by said elastomeric sleeve (22) when said skeletal frame is at or below said transition temperature.

## Patentansprüche

1. Stent zum Einsetzen in ein Körpergefäß mit einem langgestreckten rohrförmigen Körperelement (14), das eine Längsachse, ein erstes Ende, ein zweites Ende und eine langgestreckte Kammer aufweist, die sich durch das Körperelement zwischen dem ersten und dem zweiten Ende (16) erstreckt, wobei das Körperelement (14) aus einem thermischen Formspeichermaterial gebildet ist, das bei Temperaturen unter einer Übergangstemperatur relativ biegsam ist, so dass das Körperelement (14) in Richtung auf die besagte Längsachse in eine zusammengelegte Form zum Einsetzen in das Körpergefäß zusammenlegbar ist, das besagte thermische Formspeichermaterial so arbeitet, dass es das Körperelement (14) von der zusammengelegten Form in eine gedehnte Speicherform radial nach außen auf eine Temperatur auf oder über der Übergangstemperatur ausdehnt, um das Körpergefäß zu kontaktieren und daran eine radiale Kraft zu legen, das Körperelement (14) einen skelettartigen Rahmen (2) einschließt, der aus dem besagten thermischen Formspeichermaterial gebildet ist, so dass er die langgestreckte Kammer begrenzt, der skelettartige Rahmen mehrere miteinander verbundene offene Zellen (18, 20) aufweist, von denen jede zwei im Wesentlichen parallele beabstandete und gerade Seitenteile (6), die im Wesentlichen parallel zur Längsachse in der zusammengelegten Form, in der gedehnten Speicherform und während der Bewegung dazwischen verlaufen, und Endwandeinrichtungen aufweist, die zwischen den Seitenteilen (6) unter einem Winkel zur Längsachse in der gedehnten Speicherform verlaufen, und die Zellen (18, 20) entlang der Längen der geraden Seitenteile miteinander verbunden sind, **dadurch gekennzeichnet, dass** der Stent eine elastomere Hülse umfasst, die auf dem Stent angeordnet ist und damit so dehnbar ist, dass sie sich an den gedehnten Zustand des Stent anpasst, wobei die Hülse eine Vorspannung aufweist, die eine Druckkraft auf den Stent ausübt, derart, dass beim Abkühlen des Stents unter eine gewählte Übergangstemperatur die Hülse den flexiblen Stent in eine Form zwingt, die kleiner als der Stent in seinem gedehnten Zustand ist.

2. Stent nach Anspruch 1, bei dem jede Zelle (20) im ersten Bereich des skelettartigen Rahmens zwei im Wesentlichen parallele beabstandete gerade Seitenteile (6) aufweist, die um eine Strecke beabstandet sind, die im Wesentlichen gleich dem Abstand zwischen den im Wesentlichen parallelen beabstandeten Seitenteilen der Zellen (18) im zweiten Bereich des skelettartigen Rahmens sind, wobei die Endwandeinrichtungen der Zellen (20) im ersten Bereich in ihrer Länge größer als die Endwandeinrichtungen der Zellen (18) im zweiten Bereich sind.

3. Stent nach Anspruch 1, bei dem eine elastomere Hülse (22) um das besagte langgestreckte Körperelement (14) in Kontakt mit den geraden Seitenteilen (6) der Zellen (18, 20) angeordnet ist und so arbeitet, dass sie sich mit dem Körperelement (14) ausdehnt und zusammenlegt, wobei die elastomere Hülse (22) so arbeitet, dass sie dann, wenn der skelettartige Rahmen (2) die erste gedehnte Form angenommen hat, eine Druckkraft auf den skelettartigen Rahmen (2) in der ersten gedehnten Form ausübt, und weiterhin so arbeitet, dass sie dann, wenn sich der skelettartige Rahmen (2) auf oder unter der Übergangstemperatur befindet, den skelettartigen Rahmen (2) in eine zweite zusammengelegte Form zusammenlegt, die geraden Seitenteile (6) und die Endwandeinrichtungen so ausgebildet sind, dass die Seitenteile (6) parallel zur Längsachse in der ersten gedehnten Form, in der zweiten zusammengelegten Form und während der Bewegung dazwischen bleiben, und die Endwandeinrichtungen zwischen den Seitenteilen (6) unter einem Winkel zur Längsachse im ersten gedehnten Zustand verlaufen und zur Längsachse durch die elastomere Hülse (22) gedrückt werden, wenn sich der skelettartige Rahmen auf oder unter der Übergangstemperatur befindet.

4. Stent nach Anspruch 3, bei dem der erste Bereich des skelettartigen Rahmens (2) Zellen (20) einschließt, die größer als die Zellen (18) im zweiten Bereich des skelettartigen Rahmens (2) sind, wobei die Zellen (18, 20) im ersten und zweiten Bereich sich bei Temperaturen über der Übergangstemperatur so dehnen, dass sie eine radiale Kraft an die elastomere Hülse (22) legen, wobei die radiale Kraft, die von den Zellen (20) im ersten Bereich angelegt wird, kleiner als die radiale Kraft ist, die von den Zellen (18) im zweiten Bereich angelegt wird.

5. Stent nach Anspruch 1, bei dem die Zellen (18, 20) im ersten und zweiten Bereich des skelettartigen Rahmens (2) eine polygonale Form haben, **dadurch gekennzeichnet, dass** die End-' wände der Zellen (20) im ersten Bereich des skelettartigen Rahmens (2) einen größeren Winkel zur Längsachse des Körperelementes (14) als die Endwände der Zellen (18) im zweiten Bereich des skelettartigen Rahmens (2) haben, wenn sich das Körperelement (14) in der gedehnten Speicherform befindet, so dass die Zellen (20) im ersten Bereich eine kleinere radiale Kraft an das Gefäß legen, als es die Zellen (18) im zweiten Bereich tun.

6. Stent nach Anspruch 5, bei dem eine elastomere Hülse (22) um das langgestreckte Körperelement (14) in Kontakt mit den geraden Seitenteilen (6) der Zellen (18, 20) angeordnet ist und so arbeitet, dass sie sich mit dem Körperelement (14) ausdehnt und zusammenlegt, wobei die elastomere Hülse (22) so arbeitet, dass sie dann, wenn der skelettartige Rahmen (2) die erste gedehnte Form einnimmt, eine Druckkraft auf den skelettartigen Rahmen (2) in der ersten gedehnten Form ausübt, und so arbeitet, dass sie dann, wenn sich der skelettartige Rahmen (2) auf der oder unter der Übergangstemperatur befindet, den skelettartigen Rahmen (2) auf eine zweite zusammengelegte Form zusammenlegt, und die geraden Seitenteile (6) und die Endwandeinrichtungen so ausgebildet sind, dass die Seitenteile (6) parallel zur Längsachse in der ersten gedehnten Form, in der zweiten zusammengelegten Form und während der Bewegung dazwischen bleiben, während die Endwandeinrichtungen zwischen den Seitenteilen (6) unter einem Winkel zur Längsachse im ersten gedehnten Zustand verlaufen und zur Längsachse durch die elastomere Hülse (22) gedrückt werden, wenn sich der skelettartige Rahmen auf oder unter der Übergangstemperatur befindet.

## Revendications

1. Endoprothèse vasculaire à insérer dans un vaisseau corporel, incluant un corps tubulaire, allongé (14) ayant un axe longitudinal, une première extrémité, une seconde extrémité, et une cavité allongée s'étendant sur la longueur dudit corps, entre les dites première et seconde extrémités (16), le corps (14) étant formé d'un matériau à mémoire de forme thermique, qui est relativement souple à des températures inférieures à une température de transition, pour permettre audit corps (14) d'être écrasé en direction dudit axe longitudinal, en une configuration aplatie, pour l'insertion dans ledit vaisseau corporel, ledit matériau à mémoire de forme thermique agissant pour dilater ledit corps (14) de manière radiale vers l'extérieur, à partir de ladite configuration aplatie vers une configuration de mémoire expansée, en réponse à des températures égales ou supérieures à ladite température de transition, pour entrer en contact avec, et appliquer une force radiale audit vaisseau corporel, ledit corps (14) incluant une armature (2) formée dudit matériau à mémoire de forme thermique, pour définir ladite cavité allongée, l'armature incluant une pluralité de cellules ouvertes interconnectées (18, 20), chacune d'elle incluant deux portions latérales (6) droites, espacés, sensiblement parallèles, qui sont sensiblement parallèles audit axe longitudinal dans ladite configuration aplatie, ladite configuration de mémoire expansée et le mouvement entre celles-ci, et des moyens à parois d'extrémité, s'étendant entre lesdites portions latérales (6) en biais par rapport audit axe longitudinal, dans ladite configuration de mémoire expansée, lesdites cellules (18, 20) étant jointes les unes aux autres uniquement le long desdites portions latérales droites, **caractérisée en ce que** l'endoprothèse vasculaire comprend un manchon en élastomère disposé sur l'endoprothèse vasculaire et extensible avec elle pour se conformer à la condition expansée de l'endoprothèse vasculaire, le manchon ayant une précontrainte exerçant une force de compression sur l'endoprothèse vasculaire. de telle façon que, au refroidissement de l'endoprothèse vasculaire en dessous d'une température de transition sélectionnée, le manchon sollicite l'endoprothèse vasculaire flexible vers une configuration plus petite que l'endoprothèse vasculaire dans sa condition expansée.

2. Endoprothèse vasculaire selon la revendication 1 dans laquelle chaque cellule (20) dans ladite première zone de ladite armature inclut deux portions latérales droites (6), espacées, sensiblement parallèles, qui sont écartées d'une distance sensiblement égale à la distance entre les portions latérales, espacées, sensiblement parallèles des cellules (18) dans ladite seconde zone de ladite armature, les moyens de parois d'extrémités des cellules (20) dans ladite première zone étant plus longs que les moyens de parois d'extrémités des cellules (18) dans ladite seconde zone.

3. Endoprothèse vasculaire selon la revendication 1 dans laquelle un manchon en élastomère (22) est disposé autour dudit corps allongé (14), en contact avec les portions latérales droites (6) desdites cellules (18, 20), et se dilate et s'écrase avec le corps (14), ledit manchon en élastomère (22) agissant quand ladite armature (2) adopte ladite première configuration expansée, pour exercer une force de compression sur ladite armature (2) dans ladite première configuration expansée, et agissant quand ladite armature (2) est à ou en dessous de ladite température de transition, pour écraser ladite armature (2) dans la seconde configuration aplatie, lesdites portions latérales droites (6) et les moyens de parois d'extrémités étant configurés pour maintenir lesdites portions latérales (6) parallèles audit axe longitudinal dans ladite première configuration expansée, dans ladite seconde configuration aplatie et pendant le mouvement entre celles-ci, lesdits moyens de parois d'extrémités s'étendant entre lesdites portions latérales (6) en biais par rapport audit axe longitudinal dans ladite première condition expansée et étant contraints vers ledit axe longitudinal par ledit manchon en élastomère (22), quand ladite armature est à ou en dessous de ladite température de transition.

4. Endoprothèse vasculaire selon la revendication 3, dans laquelle ladite première zone de ladite armature (2) inclut des cellules (20) qui sont plus grandes que les cellules (18) dans ladite seconde zone de ladite armature (2), lesdites cellules (18, 20) dans lesdites première et seconde zones se dilatant à des températures supérieures à ladite température de transition, pour appliquer une force radiale sur ledit manchon en élastomère (22), la force radiale appliquée par les cellules (20) dans ladite première zone étant moindre que la force radiale appliquée par les cellules (18) dans ladite seconde zone.

5. Endoprothèse vasculaire selon la revendication 1 dans laquelle les cellules (18, 20) dans lesdites première et seconde zones de ladite armature (2) sont de configuration polygonale, **caractérisée en ce que** les moyens de parois d'extrémités des cellules (20) dans ladite première zone de l'armature (2) forment un plus grand angle par rapport à l'axe longitudinal dudit corps (14), que les moyens de parois d'extrémités des cellules (18) dans la seconde zone de ladite armature (2) quand ledit corps (14) est dans la configuration de mémoire expansée, de telle manière que les cellules (20) dans la première zone appliquent une force radiale moindre sur le vaisseau que ne le font les cellules (18) dans la seconde zone.

6. Endoprothèse vasculaire selon la revendication 5 dans laquelle un manchon en élastomère (22) est disposé autour dudit corps allongé (14), en contact avec les portions latérales droites (6) desdites cellules (18, 20), et se dilate et s'écrase avec le corps (14), ledit manchon en élastomère (22) agissant quand ladite armature (2) adopte ladite première configuration expansée pour exercer une force de compression sur ladite armature (2) dans ladite première configuration expansée, et agissant quand ladite armature (2) est à ou en dessous de ladite température de transition, pour écraser ladite armature (2) dans la seconde configuration aplatie, lesdites portions latérales droites (6) et les moyens de parois d'extrémités étant configurés pour maintenir lesdites portions latérales (6) parallèles audit axe longitudinal dans ladite première configuration expansée, dans ladite seconde configuration aplatie et pendant le mouvement entre celles-ci, lesdits moyens de parois d'extrémités s'étendant entre lesdites portions latérales (6), en biais par rapport audit axe longitudinal dans ladite première condition expansée, et étant contraints vers ledit axe longitudinal par ledit manchon en élastomère (22) quand ladite armature est à ou en dessous de ladite température de transition.
